(19) 

Europäisches
Patentamt
European
Patent Office
Office européen
des brevets

(11) **EP 4 755 855 A1**

(12) **EUROPEAN PATENT APPLICATION**
published in accordance with Art. 153(4) EPC

(43) Date of publication:
**10.06.2026 Bulletin 2026/24**

(21) Application number: **24849217.5**

(22) Date of filing: **31.07.2024**

(51) International Patent Classification (IPC):
*C02F 3/12* (2023.01)   *C12M 1/34* (2006.01)
*G06Q 50/00* (2024.01)   *G06T 7/60* (2017.01)
*G06T 7/254* (2017.01)

(52) Cooperative Patent Classification (CPC):
**C02F 3/12; C12M 1/34; G06Q 50/00; G06T 7/254;
G06T 7/60; Y02W 10/10**

(86) International application number:
**PCT/JP2024/027376**

(87) International publication number:
**WO 2025/028572 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **02.08.2023 JP 2023126440**

(71) Applicant: **SUMITOMO CHEMICAL COMPANY,
LIMITED**
**Tokyo 103-6020 (JP)**

(72) Inventor: **OKUDERA, Tomohiro**
**Niihama-shi, Ehime 792-0015 (JP)**

(74) Representative: **J A Kemp LLP**
**80 Turnmill Street**
**London EC1M 5QU (GB)**

(54) **COMPUTER PROGRAM, INFORMATION PROCESSING METHOD, AND INFORMATION
PROCESSING DEVICE**

(57)   Provided is a computer program capable of recognizing microorganisms with high accuracy.
The computer program causes a computer to execute processing of: acquiring an image in which a plurality of microorganisms are captured; and recognizing microorganisms in the image by applying a plurality of methods corresponding to the types or sizes of the microorganisms to the acquired image.

FIG. 8

Start

S11 Receive sludge image

S12 Analyze condition of large-sized protozoa using first method

S13 Analyze condition of small-sized protozoa using second method

S14 Analyze condition of filamentous bacteria using third method

S15 Analyze condition of flocs using fourth method

S16 Estimate health condition of activated sludge

S17 Derive proposal information

S18 Generate screen information indicating estimation result and proposal information

S19 Transmit screen information indicating estimation result and proposal information

End

# Description

Technical Field

The present invention relates to a computer program, an information processing method, and an information processing apparatus.

Background Art

Wastewater treatment systems using an activated sludge method are widely applied as treatment systems for public sewage or wastewater discharged from food factories, chemical factories, and the like. In the wastewater treatment system using the activated sludge method, wastewater to be treated is introduced into the treatment tank and subjected to an aeration treatment to supply oxygen to various types of aerobic microorganisms (activated sludge) present in the treatment tank. The organic pollutants contained in the wastewater in the treatment tank are decomposed by the action of aerobic microorganisms, thereby purifying the wastewater.

The operation status of the wastewater treatment system is greatly affected by the condition of microorganisms in the wastewater. Therefore, techniques for grasping the condition of the microorganisms have been proposed. For example, Patent Literature 1 discloses a wastewater treatment operation status evaluation device that analyzes an input image obtained by imaging microorganisms living in a water treatment tank and identifies which classification category the microorganisms belong to.

Citation List

Patent Literature:

Patent Literature 1: Japanese Patent No. 7172302

Summary

Technical Problems

However, the technique described in Patent Literature 1 has a problem that the accuracy of microorganism recognition is not sufficient. Such a problem can occur not only in wastewater treatment systems but also in any field that handles microorganisms.

It is an object of the present disclosure to provide a computer program and the like capable of recognizing microorganisms with high accuracy.

Solution to Problems

A computer program according to one aspect of the present disclosure causes a computer to execute processing of: acquiring an image in which a plurality of microorganisms are captured; and recognizing microorganisms in the image by applying a plurality of methods corresponding to types or sizes of the microorganisms to the acquired image.

An information processing method according to one aspect of the present disclosure is a method in which a computer executes processing of: acquiring an image in which a plurality of microorganisms are captured; and recognizing microorganisms in the image by applying a plurality of methods corresponding to types or sizes of the microorganisms to the acquired image.

An information processing apparatus according to one aspect of the present disclosure includes a control unit that executes processing of acquiring an image in which a plurality of microorganisms are captured and recognizing microorganisms in the image by applying a plurality of methods corresponding to types or sizes of the microorganisms to the acquired image.

Advantageous Effects of Invention

According to the present disclosure, it is possible to recognize microorganisms with high accuracy.

Brief Description of Drawings

FIG. 1 is a schematic diagram of a management system according to the present embodiment.
FIG. 2 is a block diagram showing the configuration of an information processing apparatus.
FIG. 3 is a block diagram showing the configuration of a terminal device.
FIG. 4 is a conceptual diagram showing a method for estimating the health condition of activated sludge according to a first method.
FIG. 5 is a conceptual diagram showing a method for estimating the health condition of activated sludge according to a second method.
FIG. 6 is a conceptual diagram showing a method for estimating the health condition of activated sludge according to a third method.
FIG. 7 is a conceptual diagram showing a method for estimating the health condition of activated sludge according to a fourth method.
FIG. 8 is a flowchart showing an example of a processing procedure executed by the information processing apparatus.
FIG. 9 is a schematic diagram showing an example of a display screen showing estimation results and proposal information.

Description of Embodiments

The present disclosure will be specifically described with reference to the diagrams showing embodiments thereof.

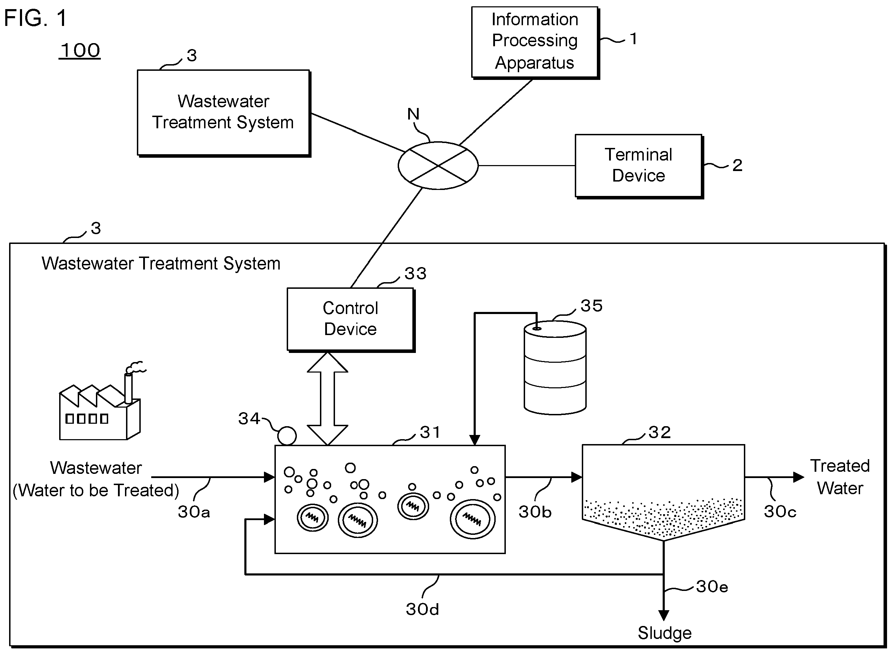

FIG. 1 is a schematic diagram of a management system 100 according to the present embodiment. In the present embodiment, a case where an object to be managed by the management system 100 is a wastewater treatment system using an activated sludge method and microorganisms in wastewater are recognized will be described as an example.

The management system 100 includes an information processing apparatus 1 as a main apparatus. The information processing apparatus 1 is communicably connected to a terminal device 2 and a plurality of wastewater treatment systems 3 through a network N such as the Internet. The wastewater treatment system 3 is a system for treating wastewater using an activated sludge method. The number of terminal devices 2 may be two or more. The number of wastewater treatment systems 3 may be one or three or more. The information processing apparatus 1 may be integrated into any of the terminal device 2 or the wastewater treatment system 3.

The information processing apparatus 1 is an apparatus capable of performing various kinds of information processing and transmitting and receiving information, and is, for example, a server computer, a personal computer, or a quantum computer. The information processing apparatus 1 is managed by, for example, a service provider of a management service provided by the management system 100. The information processing apparatus 1 acquires sludge images showing the condition of activated sludge in each wastewater treatment system 3, and estimates the condition of the activated sludge in the wastewater treatment system 3 based on the acquired sludge images. The information processing apparatus 1 provides various kinds of information including the estimated condition of the activated sludge to the terminal device 2. The information processing apparatus 1 may be communicably connected to a plurality of terminal devices 2 corresponding to a plurality of users who use the management service.

The terminal device 2 is an information processing terminal used by a user of the management service, and is, for example, a personal computer, a smartphone, or a tablet terminal. Users of the management service include, for example, organizations such as wastewater treatment providers that own the wastewater treatment system 3 and operators belonging to such organizations. The terminal device 2 may be a local computer installed in a factory including the wastewater treatment system 3.

The wastewater treatment system 3 includes a treatment tank 31 and a settling tank 32 as wastewater treatment equipment for treating wastewater using activated sludge and a control device 33 for controlling the operation of the treatment tank 31 and the settling tank 32. The activated sludge used in the wastewater treatment system 3 is a complex microbial system, and contains microorganisms such as bacteria, protozoa, and metazoa. The wastewater treated by the wastewater treatment system 3 contains organic pollutants. The wastewater is, for example, industrial wastewater or public sewage.

Wastewater (water to be treated) flows into the treatment tank 31 through a water supply line 30a. A constant amount of wastewater may be continuously sent to the treatment tank 31.

The treatment tank 31 includes an aeration means (not shown), and the water to be treated is aerobically treated by using the activated sludge present in the treatment tank 31. Using gas such as air or oxygen supplied by a blower (not shown), the treatment tank 31 diffuses the gas into the treatment tank 31 and aerates the activated sludge stored therein. The aerobic microorganisms in the activated sludge take up dissolved oxygen from the water and oxidatively decompose the organic pollutants in the water to be treated, which they have taken in as food. The water to be treated that has been treated in the treatment tank 31 is sent to the settling tank 32 through a water supply line 30b.

The settling tank 32 leaves the water to be treated, which is sent from the treatment tank 31, to stand for a predetermined period of time, so that the activated sludge is settled naturally. As a result, the water to be treated is separated into a supernatant and activated sludge by solid-liquid separation. The supernatant is discharged as treated water through a water supply line 30c. A part of the settled activated sludge is returned to the treatment tank 31 through a line 30d and recirculated. The remaining activated sludge is extracted to the outside of the system, as excess sludge, through a line 30e. The excess sludge is dewatered or dried and then incinerated and disposed of, or is recycled as a soil conditioner, civil engineering and construction materials, and the like. In addition, the settling tank 32 is not limited to the settling tank type, and may be, for example, a membrane separation device or the like.

In the treatment tank 31, a camera 34 for imaging the condition of the activated sludge in the tank is provided. The camera 34 is an example of an imaging apparatus. The camera 34 may be a high-magnification camera capable of imaging various microorganisms contained in the activated sludge at high magnification. The camera 34 generates a sludge image showing the condition of the activated sludge. The sludge image is a still image or a moving image formed by a plurality of frames of still images. The camera 34 is connected to the control device 33 by wire or wirelessly, and outputs the captured sludge image to the control device 33. The camera 34 may be provided in the settling tank 32. A plurality of cameras 34 may be provided.

In the wastewater treatment system 3, a tank 35 for storing a nutrient agent containing microorganisms as additive bacteria is also provided. The nutrient agent stored in the tank 35 is continuously added to the treatment tank 31 in predetermined amounts using, for example, a pump (not shown). The form of the nutrient agent is not limited to a formulation, but may be, for example, liquid of powder. The nutrient agent may also be added in the settling tank 32 or the water lines 30a, 30b, and 30d. The nutrient agent may be added manually.

Various detectors (not shown) for detecting temperature, flow rate, pH, DO (Dissolved Oxygen) concentration, MLSS (Mixed Liquor Suspended Solids), TOC (Total Organic Carbon), metal element concentration, and the like within the system or related to the wastewater may be provided in the wastewater treatment system 3. The detector outputs the detected value to the control device 33.

The configuration of the wastewater treatment system 3 is an example and is not limited thereto. The wastewater treatment system 3 may include, for example, a pre-treatment tank, a settling tank before treatment, and the like, which are provided before the treatment tank 31. The treatment tank 31 may be configured in a plurality of stages.

The control device 33 is a computer, and includes a control unit, a storage unit, a communication unit, and the like, all of which are not shown. The control device 33 can transmit and receive information to and from the information processing apparatus 1 through the communication unit. The control device 33 generates control signals for controlling the operations of the treatment tank 31 and the settling tank 32 and transmits the control signals to the treatment tank 31 and the settling tank 32. In addition, the control device 33 transmits a sludge image received from the camera 34 to the information processing apparatus 1. The control device 33 may be communicably connected to the information processing apparatus 1 through a relay device (not shown) such as a router or a gateway.

Since the quality or quantity of wastewater in the wastewater treatment system 3 varies greatly depending on the season or the day, the type or amount of organic pollutants contained in the wastewater also changes daily. Therefore, the ecosystem of microorganisms that make up the activated sludge also changes depending on the condition of the wastewater with which the activated sludge comes into contact.

The wastewater treatment performance of the wastewater treatment system 3 largely depends on the activated sludge. The quality or quantity of the wastewater varies greatly depending on the season or the day. Since the activated sludge is an aggregate of many types of microorganisms, changes in the quality of the wastewater with which the activated sludge comes into contact also cause changes in the ecosystem of the microorganisms that make up the activated sludge. In order to stabilize and optimize the operation of the wastewater treatment system 3, it is considered important to accurately grasp the condition of the activated sludge, including the condition of the microorganisms, and in particular, the health condition of the activated sludge. However, it is not easy to grasp the health condition of activated sludge, which is an aggregate of many types of microorganisms. The management system 100 according to the present embodiment supports the operation management of the wastewater treatment system 3 by estimating the health condition of the activated sludge in the wastewater treatment system 3 and presenting the estimated health condition to the user.

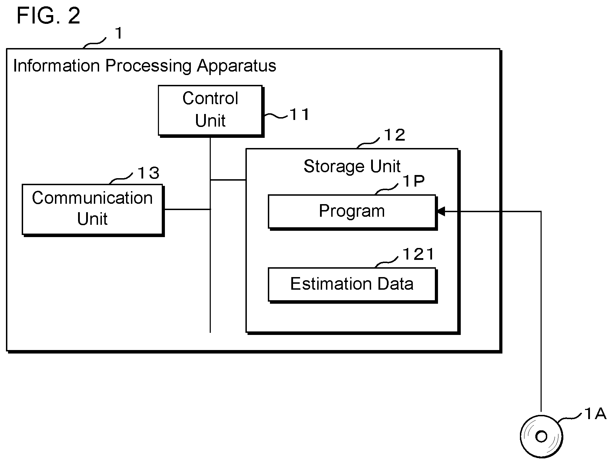

FIG. 2 is a block diagram showing the configuration of the information processing apparatus 1. The information processing apparatus 1 is a computer, and includes a control unit 11, a storage unit 12, and a communication unit 13. The information processing apparatus 1 may be a multi-computer including a plurality of computers, or may be a virtual machine virtually constructed by software.

The control unit 11 includes one or more arithmetic processing units such as a central processing unit (CPU) or a graphics processing unit (GPU). The control unit 11 controls each component by using a built-in memory such as a ROM (Read Only Memory) or a RAM (Random Access Memory), a clock, a counter, and the like, thereby executing processing. In addition, the functions of the information processing apparatus 1 may be realized by software, or some or all of the functions may be realized by hardware such as an ASIC (Application Specific Integrated Circuit) or an FPGA (Field Programmable Gate Array), for example.

The storage unit 12 includes a non-volatile memory such as a hard disk, a flash memory, or an SSD (Solid State Drive), for example. The storage unit 12 may be an external storage device connected to the information processing apparatus 1. The storage unit 12 stores various computer programs and data that the control unit 11 refers to. The storage unit 12 stores a program 1P for causing a computer to execute processing related to estimation of the health condition of activated sludge and estimation data 121 necessary for executing the program 1P. The estimation data 121 includes, for example, sludge images received from each wastewater treatment system 3, detection models or numerical expressions for performing microbial state analysis described below, recommended information tables, various analysis results, and the like.

A computer program (computer program product) including the program 1P may be provided by a non-transitory recording medium 1A on which the computer program is recorded in a readable manner. The storage unit 12 stores a computer program read from the recording medium 1A by a reading device (not shown). The recording medium 1A is, for example, a magnetic disk, an optical disk, or a semiconductor memory. Alternatively, a computer program may be downloaded from an external server connected to the communication network and stored in the storage unit 12. The program 1P may be a single computer program or may be configured by a plurality of computer programs, and may be executed on a single computer or on a plurality of computers interconnected through a communications network.

The communication unit 13 includes a communication device to realize communication through the network N. The control unit 11 transmits and receives data to and from the terminal device 2 and the control device 33 through the communication unit 13.

The configuration of the information processing apparatus 1 is not limited to the above example, and may include, for example, an operation unit for receiving the user's operation, a display unit for displaying images, and the like.

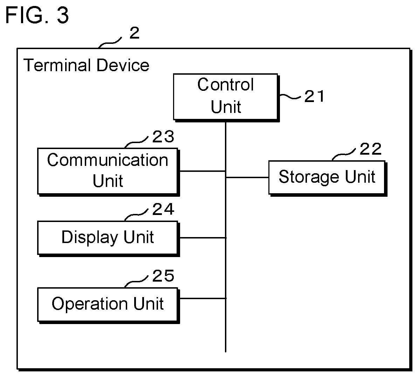

FIG. 3 is a block diagram showing the configuration of the terminal device 2. The terminal device 2 is a computer, and includes a control unit 21, a storage unit 22, a communication unit 23, a display unit 24, and an operation unit 25.

The control unit 21 includes one or more arithmetic processing units such as a CPU, an MPU, or a GPU. The control unit 21 controls each component by using a built-in memory such as a ROM or a RAM, a clock, a counter, and the like, thereby executing processing.

The storage unit 22 includes a non-volatile memory such as a hard disk, a flash memory, or an SSD, for example. The storage unit 22 stores various computer programs and data that the control unit 21 refers to. The storage unit 22 stores a computer program for causing a computer to execute processing related to receiving the estimation result of the health condition of activated sludge.

The communication unit 23 includes a communication device to realize communication through the network N. The control unit 21 transmits and receives data to and from the information processing apparatus 1 through the communication unit 23.

The display unit 24 includes a display device such as a liquid crystal display or an organic EL (Electro Luminescence) display. The display unit 24 displays the estimation results and the like received from the information processing apparatus 1 in accordance with instructions from the control unit 21.

The operation unit 25 is an interface for receiving the user's operation. The operation unit 25 includes, for example, a keyboard, a mouse, a touch panel device with a built-in display, a speaker, a microphone, and the like. The operation unit 25 receives an operation input from the user and transmits a control signal corresponding to the operation content to the control unit 21.

The information processing apparatus 1 according to the present embodiment automatically executes processing for recognizing the condition of microorganisms and estimating the health condition of activated sludge based on sludge images acquired from the wastewater treatment system 3. The health condition of activated sludge is estimated by extracting various microorganisms from the sludge image through image analysis of the sludge image and recognizing the number, size, type, and other conditions of the extracted microorganisms.

The condition of microorganisms is analyzed by classifying the microorganisms that make up activated sludge into a plurality of groups and applying different algorithms for each group. Microorganisms are classified based on one or both of the type and size of the microorganisms. In this specification, the microorganisms to be recognized include not only microorganisms themselves such as bacteria and protozoa, but also aggregates of microorganisms such as flocs.

In the present embodiment, four types of methods, from the first method to the fourth method, are used. The first method is a method for analyzing the condition of protozoa having a size equal to or greater than a predetermined value. The second method is a method for analyzing the condition of protozoa having a size less than a predetermined value. The third method is a method for analyzing the condition of bacteria, particularly filamentous bacteria. The fourth method is a method for analyzing the condition of flocs. Hereinafter, detailed explanation will be given in order from the first method.

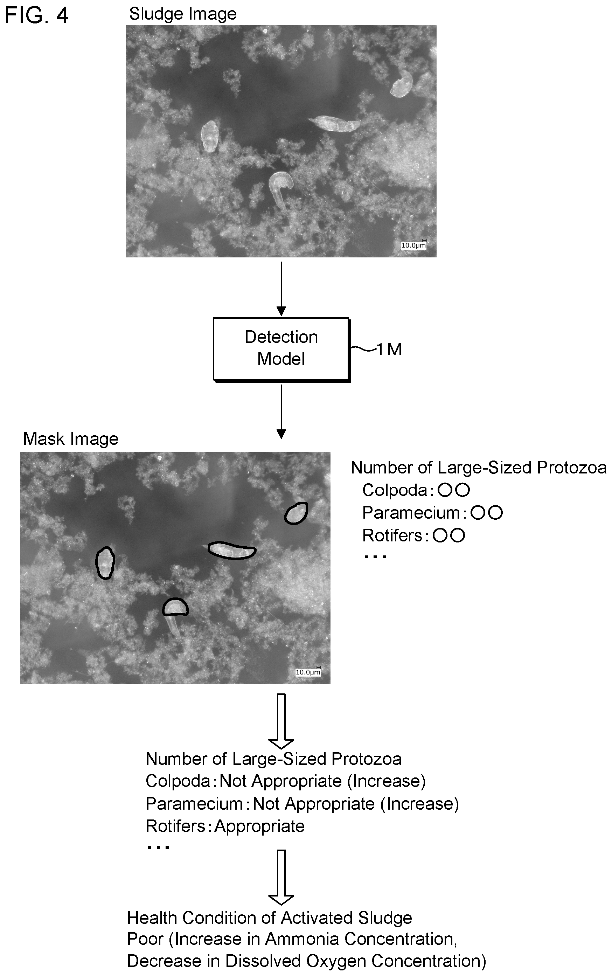

FIG. 4 is a conceptual diagram showing a method for estimating the health condition of activated sludge according to the first method. The information processing apparatus 1 analyzes the condition of protozoa having a size equal to or greater than a predetermined value by applying the first method. The protozoa that are the target of the first method are, for example, protozoa having a size of 100 $\mu$m or more. Hereinafter, protozoa having a size equal to or greater than a predetermined value will also be referred to as large-sized protozoa. Examples of large-sized protozoa include Colpoda, Paramecium, Rotifers, and Archera.

In the first method, the object region of large-sized protozoa in the sludge image is detected (recognized) using a detection model 1M. The detection model 1M is a learning model generated by machine learning. The detection model 1M is expected to be used as a program module that configures a part of artificial intelligence software,

The detection model 1M receives a sludge image as its input and outputs information indicating an object region in the sludge image. The detection model 1M is, for example, Mask R-CNN (Regions with Convolutional Neural Networks). The detection model 1M recognizes whether or not each pixel in the input image corresponds to an object region, on a pixel-by-pixel basis, by using an image recognition technique using instance segmentation. When a plurality of detection target classes (large-sized protozoa) are captured, the detection model 1M recognizes these separately.

Mask R-CNN is a variant of Faster R-CNN, which is mainly used for object detection, and is a model obtained by adding a deconvolution layer to Faster R-CNN. The detection model 1M includes a CNN and an RPN (Region Proposal Network), and inputs the feature quantities of the input data extracted by the CNN and the coordinate range of the target object extracted by the RPN to the deconvolution layer to perform object class identification and region extraction. The detection model 1M finally generates a mask image that masks the image region corresponding to the object in the image.

FIG. 4 shows an example of a mask image in which a plurality of large-sized protozoa are detected as objects. A mask indicating the detection result is applied to an image region corresponding to each object. In the example of FIG. 4, only the boundaries of the image regions corresponding to the objects are shown by thick lines. The mask image allows the type, position, and shape of the protozoa to be visually recognized. The mask image may display the detected types of protozoa in an identifiable manner, for example, by applying a mask of different colors for each object class, that is, for each type of protozoa. The detection model 1M may be configured to output the position (region) of protozoa using a bounding box.

The detection model 1M can be generated by preparing training data, in which sludge images including objects are associated with labels indicating the position and type of each object, and training an untrained neural network by machine learning using the training data.

Specifically, the information processing apparatus 1 inputs the sludge image included in the training data to the input layer of the detection model 1M, and acquires the position and type of the object output from the output layer after calculation processing in the intermediate layer. The information processing apparatus 1 compares the position and type of the object output from the output layer with the position and type of the object included in the training data, and optimizes parameters such as the weights (connection coefficients) between neurons so that the position and type of the object output from the output layer become closer to the correct values. The method for optimizing the parameters is not particularly limited, but for example, the information processing apparatus 1 optimizes various parameters using a backpropagation method.

The detection model 1M may be a 3D-CNN that handles three-dimensional input data. In this case, the detection model 1M can receive, as its input, an image set including a plurality of frames of sludge images that are consecutive in a predetermined unit time, and simultaneously output, for each of the plurality of frames of sludge images, images in which the image regions corresponding to objects are labeled. According to the above configuration, since objects can be detected by taking into consideration the preceding and following frame images that are consecutive in time series, it is possible to improve detection accuracy.

Once the learning is completed, the detection model 1M is constructed that has been trained to be able to appropriately recognize objects in sludge images. Using the detection model 1M, it is possible to obtain detection results for large-sized protozoa in sludge images.

The configuration of the detection model 1M is not limited to the example shown in FIG. 4. The detection model 1M only needs to be able to identify large-sized protozoa included in the sludge image. The detection model 1M may be a model based on other learning algorithms such as a U-Net, an RNN (Recurrent Neural Network), an SVM (Support Vector Machine), and a decision tree. The detection model 1M may be configured to detect large-sized protozoa included in the sludge image without distinguishing between types of protozoa.

The information processing apparatus 1 counts the large-sized protozoa detected by the detection model 1M and determines whether or not the number of large-sized protozoa is appropriate. The determination of whether or not the number of protozoa is appropriate is performed, for example, by determining whether or not the amount of increase in the number of protozoa calculated based on a comparison between the previous detection count and the current detection count is within an allowable range of the amount of increase set in advance. When the amount of increase in the number of protozoa is within the allowable range of increase set in advance, it is determined that the number of protozoa is appropriate. When the amount of increase in the number of protozoa is not within the allowable range of increase set in advance, it is determined that the number of protozoa is not appropriate and the number of protozoa is increasing. The determination of whether or not the number of protozoa is appropriate may be performed by determining whether or not the current detection count is within an allowable range of detection count set in advance. The determination of whether or not the number of large-sized protozoa is appropriate may be performed for each type of large-sized protozoa.

When the numbers of all types of large-sized protozoa are appropriate, it can be determined that the health condition of the activated sludge is good, that is, the activated sludge is healthy. When the number of at least one type of large-sized protozoa is not appropriate, it can be determined that the health condition of the activated sludge is poor, that is, the activated sludge is not healthy.

When the health condition of the activated sludge is poor, details of the abnormality may be further determined. The determination of the details of the abnormality can be performed based on the detection results for each type of protozoa. The cause of an increase in the number of protozoa varies depending on the type of protozoa. For example, an increase in the number of Colpoda is considered highly likely to be caused by an increase in the ammonia concentration in the wastewater. Therefore, if the number of Colpoda is not appropriate, the health condition can be determined to be poor (increase in ammonia concentration). Alternatively, an increase in the number of Paramecium is considered highly likely to be caused by a decrease in the dissolved oxygen concentration in the wastewater. Therefore, if the number of Paramecium is not appropriate, the health condition can be determined to be poor (decrease in dissolved oxygen concentration).

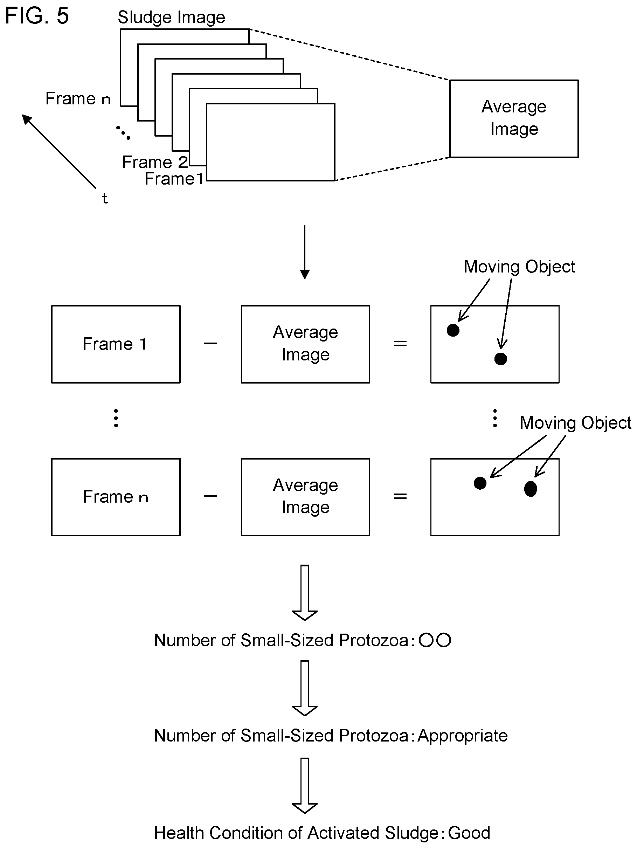

FIG. 5 is a conceptual diagram showing a method for estimating the health condition of activated sludge according to the second method. The information processing apparatus 1 analyzes the condition of protozoa having a size less than a predetermined value by applying the second method. The protozoa that are the target of the second method are, for example, protozoa having a size of less than 100 μm. Hereinafter, protozoa having a size less than a predetermined value will also be referred to as small-sized protozoa. As an example of small-sized protozoa, Bodo can be mentioned.

In the second method, small-sized protozoa in sludge images are detected by performing moving-object detection from a plurality of frames of sludge images. Specifically, as shown in FIG. 5, the information processing apparatus 1 generates an average image by averaging all the frames using a moving average of a moving image for a predetermined unit time, that is, a plurality of frames consecutive in chronological order. Then, moving objects are detected from the difference between the generated average image and each frame. The moving object detection conditions may be to detect only objects less than a predetermined value (for example, objects whose maximum lengths in the object region are less than 100 μm). In addition, the moving object detection method is not particularly limited, and the moving object may be detected by calculating the difference between adjacent frames.

The information processing apparatus 1 counts the detected small-sized protozoa based on the moving object detection result. The information processing apparatus 1 may determine the final number of small-sized protozoa by calculating the average value, mode, median, or the like for all frames based on the number of small-sized protozoa determined for each frame.

The information processing apparatus 1 determines whether or not the number of small-sized protozoa is appropriate based on the calculated number of small-sized protozoa. Whether or not the number of small-sized protozoa is appropriate can be determined based on the number of protozoa or the amount of increase in the number of protozoa, as in the case of large-sized protozoa. In addition, details of the determination may differ depending on each method, for example, such that in the first method, the appropriateness of the number of protozoa is determined based on the trend of change in the amount of increase in the number of protozoa, and in the second method, the appropriateness is determined based on the number of protozoa.

When the number of small-sized protozoa is appropriate, it can be determined that the health condition of the activated sludge is good. When the number of small-sized protozoa is not appropriate, it can be determined that the health condition of the activated sludge is poor.

An increase in the number of protozoa such as Bodo is considered highly likely to be caused by an increase in sludge loading. Therefore, when the number of small-sized protozoa is not appropriate and the number of small-sized protozoa is increasing, the health condition may be specifically determined to be poor (an increase in sludge loading).

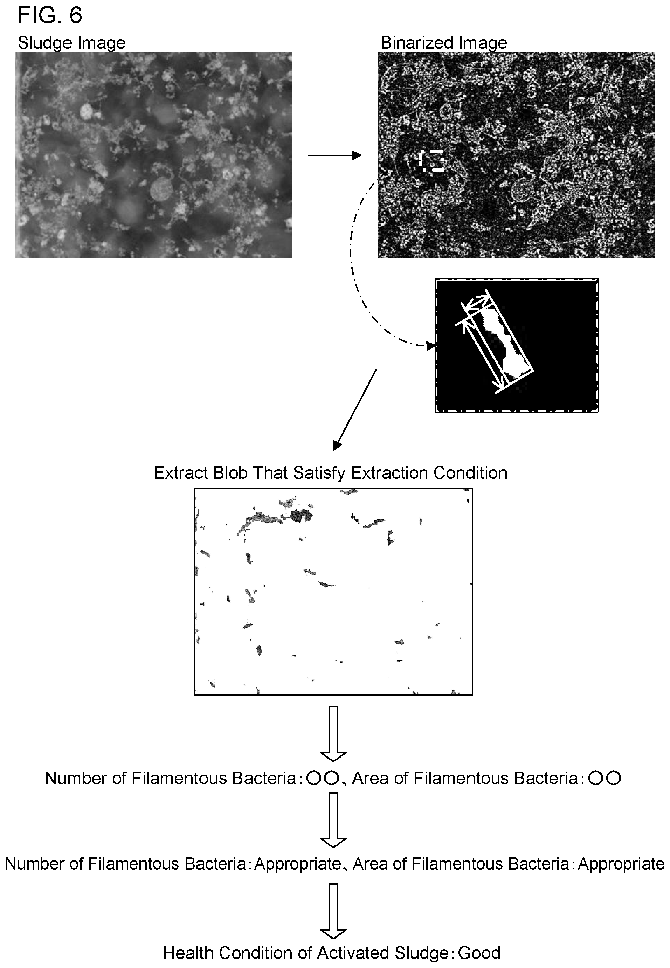

FIG. 6 is a conceptual diagram showing a method for estimating the health condition of activated sludge according to the third method. The information processing apparatus 1 analyzes the condition of filamentous bacteria by applying the third method. In addition, the lower image of the binarized image shown at the upper right of FIG. 6 is an enlarged view of a partial region of the upper binarized image.

In the third method, filamentous bacteria in the sludge image are detected by performing binarization processing on the sludge image. Specifically, the information processing apparatus 1 performs binarization processing on the sludge image converts the pixel values of the sludge image into 0 (black) or 1 (white). The method of binarization processing is not particularly limited. For example, the brightness value (pixel value) of a pixel is compared with a threshold value calculated using a predetermined method, and the pixel value is set to 1 when the pixel value is equal to or greater than the threshold value.

By analyzing the binarized image obtained by the binarization processing, a region with a pixel value equal to or greater than the threshold value is specified as a blob region. A predetermined feature quantity corresponding to the specified blob region is calculated, and it is determined whether or not each calculated feature quantity satisfies the threshold conditions set in advance. Examples of the feature quantity include area, circularity, aspect ratio, maximum height, and maximum width.

The aspect ratio is the ratio of the height to the width of a blob region. In the present embodiment, the aspect ratio is defined as the ratio (width/height) of the width to the height of the blob region. In the lower image of the binarized image shown in FIG. 6, the long side of the white rectangular region represents the height, and the short side represents the width.

The circularity is expressed by the following equation, where the area of the blob region is S and the perimeter of the blob region is L.

$$\text{Circularity} = 4\pi S/L^2$$

Examples of the threshold conditions for each feature quantity include: the area is 50 μm$^2$ or more; the circularity is 0.7 or less; the aspect ratio exceeds 2.5; the maximum height is 100 μm or less; and the maximum width is 100 μm or less. It is determined whether or not each calculated feature quantity satisfies the threshold conditions, and blob regions that satisfy the threshold conditions for all feature quantities are finally extracted as filamentous bacteria. In addition, when a predetermined number or more of feature quantities for a blob region satisfy the threshold conditions, the blob region may be recognized as a filamentous bacterium. In addition, the feature quantity and the threshold conditions for the feature quantity are not limited to the above examples, and any feature quantity and threshold conditions may be used as long as filamentous bacteria can be recognized from the binarized image.

The information processing apparatus 1 counts the extracted filamentous bacteria and determines whether or not the number of filamentous bacteria is appropriate. Whether or not the number of filamentous bacteria is appropriate can be determined based on the number of filamentous bacteria or the amount of increase in the number, as in the case of protozoa. When the number of filamentous bacteria is appropriate, it can be determined that the health condition of the activated sludge is good. When the number of filamentous bacteria is not appropriate and the number of filamentous bacteria is increasing, it can be determined that the health condition of the activated sludge is poor.

An increase in the number of filamentous bacteria is considered highly likely to be caused by a decrease in the dissolved oxygen concentration in the wastewater or a shortage of nutrient sources (for example, nitrogen sources and phosphorus sources). Therefore, when the number of filamentous bacteria is not appropriate, the health condition may be specifically determined to be poor (a decrease in dissolved oxygen concentration or a shortage of nutrient sources).

In the determination processing of the first to third methods, instead of or in addition to the number of protozoa or filamentous bacteria in the detected sludge image, it may be determined whether or not the area of the detected protozoa or filamentous bacteria is appropriate. When the total area of the regions corresponding to the detected protozoa or filamentous bacteria or the amount of increase in the total area is within an allowable range, it can be determined that the area of the protozoa or filamentous bacteria is appropriate and the health condition of the activated sludge is good. When the area of the protozoa or filamentous bacteria is not appropriate, it can be determined that the protozoa or filamentous bacteria are increasing and the health condition of the activated sludge is poor.

In addition, in the determination processing of the first to third methods, since the number and area of microorganisms depend on the amount of activated sludge itself, the number and area may be calculated relatively based on the amount of activated sludge in the sludge image. In this case, the region of activated sludge in the sludge image is specified by analyzing the sludge image, and the area of the identified activated sludge region is calculated. Based on the number of protozoa or filamentous bacteria with respect to the calculated area of the activated sludge region and the area of the protozoa or filamentous bacteria with respect to the area of the activated sludge region, the appropriateness of the number and area is determined.

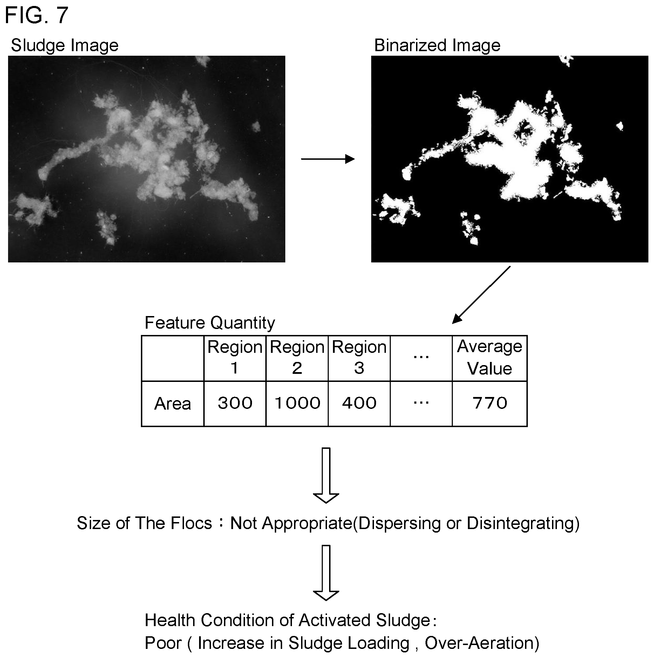

FIG. 7 is a conceptual diagram showing a method for estimating the health condition of activated sludge according to the fourth method. The information processing apparatus 1 analyzes the condition of flocs by applying the fourth method.

In the fourth method, flocs in a sludge image are detected by performing binarization processing on the sludge image. Specifically, the information processing apparatus 1 binarizes the sludge image using a threshold value calculated using a predetermined method, and converts the pixel values of the sludge image into 0 or 1. For example, when the pixel value is equal to or greater than the threshold value, the pixel value is set to 1. The threshold value for the binarization processing in the fourth method may be different from the threshold value for the binarization processing in the third method.

In the fourth method, a sludge image may be filtered to extract predetermined spatial frequency components, and the extracted spatial frequency components may be binarized. The filtering process is performed using, for example, fast Fourier transformation (FFT). The method of binarizing the sludge image is not limited to the above example.

By analyzing the binarized image obtained by the binarization processing, a region with a pixel value equal to or greater than the threshold value is specified as a blob region corresponding to flocs. The information processing apparatus 1 calculates a predetermined feature quantity for each specified blob region, and specifies the condition of floc formation based on the calculated feature quantity for each blob region. Examples of the feature quantity include area, circularity, and color.

The information processing apparatus 1 calculates, for example, a statistical value (for example, an average value, a total value, or a median) of the calculated feature quantities for each feature quantity, and determines whether or not the size of the flocs is appropriate based on the obtained statistical value. For example, when the amount of decrease in the average area value of the flocs calculated based on a comparison between the previous average value of the area and the current average value of the area is within an allowable range of decrease set in advance, it can be determined that the size of the flocs is appropriate. When the amount of decrease in the average value of the area of the flocs is not within the allowable range set in advance, it can be determined that the size of the flocs is not appropriate and the flocs are dispersing or disintegrating. The determination of whether or not the size of the flocs is appropriate may be performed by determining whether or not the current statistical value (for example, the average value of the area) is within an allowable range of statistical values set in advance. Alternatively, the information processing apparatus 1 may determine the color of the flocs in the extracted floc region by using a known color analysis method, and determine whether or not the determined color of the flocs is appropriate.

When the size of the flocs is appropriate, it can be determined that the health condition of the activated sludge is good. When the size of the flocs is not appropriate and the flocs are dispersing or disintegrating, it can be determined that the health condition of the activated sludge is poor.

The dispersion or disintegration of flocs is considered highly likely to be caused by an increase in sludge loading or by over-aeration. Therefore, when the size of the flocs is not appropriate, the health condition may be specifically determined to be poor (an increase in sludge loading or over-aeration).

The image analysis methods in the first to fourth methods described above are merely examples, and the image analysis methods and the types and sizes of analysis targets using such methods are not limited to the above examples. The health condition can be estimated by combining appropriate analysis methods as long as microorganisms to be detected can be detected from sludge images and at least two or more methods corresponding to the types of microorganisms to be detected are used.

The information processing apparatus 1 according to the present embodiment further generates proposal information for optimizing the operation of the wastewater treatment system 3 according to the estimation result of the health condition of the activated sludge. The proposal information includes, for example, at least one of a proposal regarding the operating conditions of the wastewater treatment system 3 and a proposal regarding the conditions for adding nutrients.

The information processing apparatus 1 stores in advance in the storage unit 12, for example, a proposal information table that associates the health condition of activated sludge with proposal information, and generates proposal information using the proposal information table. The information processing apparatus 1 specifies proposals for operating conditions or addition conditions that are associated with details of the estimated poor health condition by referring to the proposal information table.

For example, in the case of a poor condition due to an increase in Colpoda (an increase in ammonia concentration), dilution of the wastewater is proposed. In the case of a poor condition due to an increase in Paramecium (a decrease in dissolved oxygen concentration), an increase in the aeration flow rate is proposed. In the case of a poor condition (an increase in sludge loading) due to an increase in small-sized protozoa, suppression of the treatment flow rate, an increase in MLSS, and the like are proposed. In the case of a poor condition due to an increase in filamentous bacteria (a decrease in dissolved oxygen concentration or shortage of nutrients), an increase in the aeration flow rate, addition of nutrients, and the like are proposed. In the case of a poor condition due to dispersion or disintegration of flocs (an increase in sludge loading or over-aeration), suppression of the treatment flow rate, an increase in MLSS, an increase in the aeration flow rate, and the like are proposed.

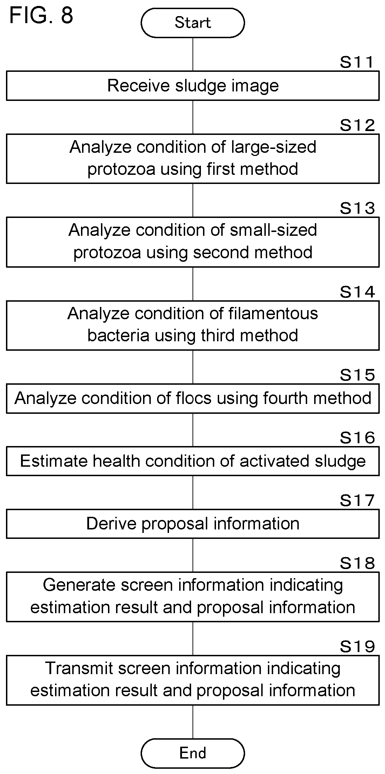

FIG. 8 is a flowchart showing an example of a processing procedure executed by the information processing apparatus 1. The processing in the following flowchart is executed by the control unit 11 in accordance with the program 1P stored in the storage unit 12 of the information processing apparatus 1.

The control unit 11 of the information processing apparatus 1 receives a sludge image of the wastewater treatment system 3 from the control device 33 (step S11), and stores the received sludge image in the storage unit 12. The sludge image is associated with identification information for identifying the control device 33 or the wastewater treatment system 3.

The control unit 11 analyzes the condition of large-sized protozoa using the first method based on the acquired sludge image (step S12). In step S12, the control unit 11 inputs the sludge image to the detection model 1M and acquires a mask image output from the detection model 1M in which large-sized protozoa are detected. The mask image is, for example, information indicating large-sized protozoa for each type. The control unit 11 determines the types of large-sized protozoa and the number for each type in the sludge image, and determines, for each type, whether or not the number of large-sized protozoa is appropriate based on the determined number of large-sized protozoa of each type.

The control unit 11 analyzes the condition of small-sized protozoa using the second method based on the acquired sludge image (step S13). In step S13, the control unit 11 detects small-sized protozoa in the sludge image by detecting moving objects from a plurality of time-series frames of sludge image, and calculates the number of small-sized protozoa. The control unit 11 determines whether or not the number of small-sized protozoa is appropriate based on the detected number of small-sized protozoa.

The control unit 11 analyzes the condition of filamentous bacteria using the third method based on the acquired sludge image (step S14). In step S14, the control unit 11 recognizes blob regions by performing binarization processing on the sludge image, and detects filamentous bacteria in the sludge image by specifying a blob region whose feature quantity satisfies predetermined threshold conditions among the recognized blob regions, thereby calculating the number of filamentous bacteria. The control unit 11 determines whether or not the number of filamentous bacteria is appropriate based on the detected number of filamentous bacteria.

The control unit 11 analyzes the condition of flocs using the fourth method based on the acquired sludge image (step S15). In step S15, the control unit 11 detects blob regions representing flocs by performing binarization processing on the sludge image. The control unit 11 determines whether or not the size of the flocs is appropriate based on the statistical values of predetermined feature quantities related to the detected blob region.

The control unit 11 estimates the health condition of the activated sludge based on the analysis results of the conditions of large-sized protozoa, small-sized protozoa, filamentous bacteria, and flocs (step S16). The health condition of the activated sludge may be estimated for each group of microorganisms or for each type in each group. Specifically, the control unit 11 determines whether the health condition of the activated sludge is good or poor based on whether or not the number of large-sized protozoa is appropriate, whether or not the number of small-sized protozoa is appropriate, whether or not the number of filamentous bacteria is appropriate, and whether or not the size of the flocs is appropriate. In addition to determining whether the health condition is good or poor, the control unit 11 may determine details when the health condition is poor. The control unit 11 stores the analysis results of the condition of various microorganisms and the determination results of the health condition in the storage unit 12.

The control unit 11 derives proposal information corresponding to the estimated health condition of the activated sludge by referring to the proposal information table of the estimation data 121 (step S17).

The control unit 11 generates screen information indicating the estimation result of the health condition of the activated sludge and the derived proposal information (step S18). The control unit 11 transmits the generated screen information indicating the estimation result and the proposal information to the terminal device 2 of the user of the wastewater treatment system 3 (step S19). The control unit 11 may specify the terminal device 2 to which the screen information is to be transmitted based on the identification information of the control device 33 or the wastewater treatment system 3 associated with the sludge information. The control unit 11 ends the series of processes.

The information processing apparatus 1 repeatedly executes the above-described processing each time a sludge image is acquired. The information processing apparatus 1 preferably acquires a sludge image at predetermined intervals and executes the above-described processing in real time, thereby enabling early notification of deterioration in the health condition.

Although an example in which the information processing apparatus 1 executes a series of processes has been described above, the entity that performs each process is not limited. Some or all of the processes in the flowchart may be executed by the terminal device 2 or the control device 33, for example.

In the above-described process, when it is determined that the health condition of the activated sludge is poor, the control unit 11 may transmit an alert notifying that the health condition is poor to the terminal device 2 or the control device 33. The estimation result and the proposal information may be transmitted only when the health condition of the activated sludge is determined to be poor.

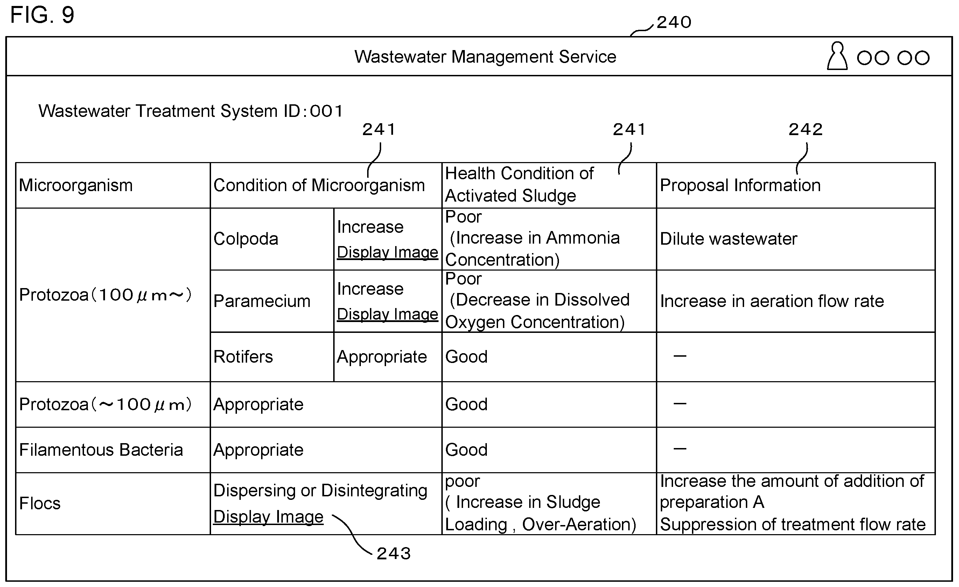

FIG. 9 is a schematic diagram showing an example of a display screen 240 that displays the estimation results and the proposal information. The control unit 21 of the terminal device 2 displays the display screen 240 shown in FIG. 9 on the display unit 24 based on the screen information received from the information processing apparatus 1.

The display screen 240 includes, for example, an estimation result field 241 for displaying estimation results and a proposal information field 242 for displaying proposal information. The estimation result field 241 and the proposal information field 242 display the estimation results and the proposal information for each type and size of microorganism. The estimation result field 241 displays the determination results of the condition of microorganisms and the determination results of the health condition of the activated sludge. The proposal information field 242 displays proposals of various conditions corresponding to the health condition of the activated sludge.

The information processing apparatus 1 displays, in the estimation result field 241, the condition of microorganisms derived by each method (for example, the presence or absence of an increase or the presence or absence of dispersion or disintegration) and whether the health condition of the activated sludge is good or poor based on the condition of microorganisms. In addition, the proposal information specified based on the health condition of the activated sludge is displayed in the proposal information field 242. When the health condition is good, proposal information may not be generated, or proposal information may be presented as unchanged.

The information processing apparatus 1 may also display, for microorganisms determined to have an inappropriate condition, an image showing the condition of the microorganisms on the display screen 240. In the example shown in FIG. 9, a hyperlink 243 for accessing an image showing the condition of the microorganisms is displayed on the display screen 240. When it is determined that the condition of any microorganism is not appropriate, the information processing apparatus 1 generates an image showing the condition of the microorganism based on information stored in the storage unit 12, and displays the generated image or a hyperlink to the image on the display screen 240. The image showing the condition of the microorganism is an image that presents the condition of the microorganism in a visually recognizable manner, and is, for example, an image subjected to processing such as superimposing a mask, a frame, or the like representing microorganism detection results on a sludge image. The image showing the condition of the microorganism may be a mask image generated by the detection model 1M, or may be the sludge image itself.

According to the present embodiment, the condition of microorganisms and the health condition of activated sludge can be automatically estimated based on sludge images. The condition of microorganisms and the health condition of activated sludge are estimated using different algorithms according to the group to which the microorganisms belong. By applying different algorithms for each group to which the microorganisms belong to recognize the microorganisms in the sludge image, the accuracy of recognizing the microorganisms is improved. Since microorganisms in the image can be detected with high accuracy, the accuracy of recognizing the number of microorganisms is improved. In addition, according to the present embodiment, microorganisms can be observed at a fixed magnification, which eliminates the need to change magnification, shortens observation time, and enables efficient execution of analysis processing at a low magnification.

By determining the algorithm to be applied according to the characteristics of the microorganism group to be analyzed or the detection content, it is possible to perform accurate analysis while reducing the overall processing load. In the case of protozoa, by using the first and second methods according to the size, it becomes possible to detect relatively large protozoa by distinguishing between different types of protozoa, and to detect relatively small protozoa without distinguishing between different types of protozoa. In the case of filamentous bacteria, by setting extraction conditions using feature quantities, filamentous bacteria with characteristic shapes can be efficiently detected.

For example, by quantitatively evaluating the condition of microorganisms, such as the number and area of microorganisms, the condition of the microorganisms can be accurately grasped. Since the health condition of the activated sludge is estimated based on the quantitative evaluation results of the microorganism condition, the estimation accuracy is improved. It is possible to realize automatic estimation of the health condition of activated sludge without depending on the ability of the operator.

According to the present embodiment, since it is possible to provide proposal information according to the health condition of the activated sludge, it is possible to realize optimization of the operation management of the wastewater treatment system 3.

By providing the user with a screen displaying the estimation results of the health condition or the proposal information, the user can clearly grasp the state of the wastewater treatment system 3 through the screen.

The wastewater condition in the wastewater treatment system 3 varies on a daily basis. In addition, since the environment of the wastewater treatment system 3 differs depending on the facility, the condition of the activated sludge differs for each wastewater treatment system 3. In this system, by using sludge images showing the actual condition of wastewater collected from each wastewater treatment system 3, it is possible to estimate the health condition reflecting the state of the wastewater treatment system 3 for each wastewater treatment system 3.

The computer program, the information processing method, and the information processing apparatus can also be applied to purposes other than wastewater treatment systems. For example, the information processing method and the information processing apparatus can be applied to the cultivation of feed for fish. When cultivating rotifers as food, it is desirable to co-cultivate these with other microorganisms. However, a large number of Vorticella cause the rotifers to weaken. For this reason, it is useful to apply this method to appropriately count each microorganism and manage the cultivation condition.

Regarding the above-described embodiments, the following supplementary notes are further disclosed.

(Supplementary Note 1)

A computer program causing a computer to execute processing of: acquiring an image in which a plurality of microorganisms are captured; and recognizing microorganisms in the image by applying a plurality of methods corresponding to types or sizes of the microorganisms to the acquired image.

(Supplementary Note 2)

The computer program according to supplementary note 1, wherein recognizing the microorganisms in the image includes recognizing the number of microorganisms in the image or the types of microorganisms in the image.

(Supplementary Note 3)

The computer program according to supplementary note 1 or 2, wherein the plurality of methods include a first method corresponding to microorganisms having a first size and a second method corresponding to microorganisms having a second size smaller than the first size.

(Supplementary Note 4)

The computer program according to any one of supplementary notes 1 to 3, wherein the plurality of methods include a first method for detecting microorganisms having a first size using an object detection model and a second method for detecting microorganisms having a second size smaller than the first size by detecting a moving object from the time-series image.

(Supplementary Note 5)

The computer program according to supplementary note 4, wherein the image is configured by a plurality of frames in chronological order, and in the second method, the moving object is detected based on a difference between each frame and an average image obtained by averaging a plurality of frames over a predetermined period of time.

(Supplementary Note 6)

The computer program according to any one of supplementary notes 1 to 5, wherein the image is a sludge image obtained by imaging sludge in a wastewater treatment system using an activated sludge method.

(Supplementary Note 7)

The computer program according to supplementary note 6, wherein protozoa as the microorganisms in the sludge image are recognized using the plurality of methods, and a condition of activated sludge is estimated based on conditions of the recognized protozoa in the sludge image.

(Supplementary Note 8)

The computer program according to supplementary note 7, wherein proposal information regarding operating conditions of the wastewater treatment system is derived based on the estimated condition of the activated sludge.

(Supplementary Note 9)

The computer program according to any one of supplementary notes 6 to 8, wherein the plurality of methods include a third method corresponding to filamentous bacteria and a fourth method corresponding to flocs.

(Supplementary Note 10)

The computer program according to any one of supplementary notes 6 to 9, wherein information indicating the estimated condition of the activated sludge is transmitted to a user of the wastewater treatment system.

(Supplementary Note 11)

The computer program according to any one of supplementary notes 6 to 10, wherein screen information displaying the condition of the microorganisms and the condition of the activated sludge in association with each other is generated for each type or size of the microorganisms.

The embodiments disclosed herein should be considered as illustrative in all respects and not restrictive. The technical features described in the respective embodiments can be combined with each other, and it is intended that the scope of the present invention includes all modifications within the scope of the claims and equivalents thereof.

The sequences shown in the respective embodiments are not limited, and as long as there is no contradiction, each processing procedure may be executed in a different order, and a plurality of processes may be executed in parallel. The entity that performs each process is not limited, and the processing of each device may be executed by another device as long as there is no contradiction.

The features described in the respective embodiments can be combined with each other. In addition, the independent claims and the dependent claims described in the claims can be combined with each other in all combinations, regardless of the citation form. In addition, although the form of multiple claims that cite two or more other claims (multi-claim form) is used in the scope of claims, the present invention is not limited thereto. A form of multi-claim (multiple claim) that cites at least one multi-claim can also be used.

Reference Signs List

100   management system
1   information processing apparatus
11   control unit
12   storage unit
13   communication unit
1P   program
1A   recording medium
2   terminal device
21   control unit
22   storage unit
23   communication unit
24   display unit
25   operation unit
3   wastewater treatment system

**Claims**

1. A computer program causing a computer to execute processing of:

   acquiring an image in which a plurality of microorganisms are captured; and
   recognizing microorganisms in the image by applying a plurality of methods corresponding to types or sizes of the microorganisms to the acquired image.

2. The computer program according to claim 1, wherein recognizing the microorganisms in the image includes recognizing the number of microorganisms in the image or the types of microorganisms in the image.

3. The computer program according to claim 1 or 2, wherein the plurality of methods include a first method corresponding to microorganisms having a first size and a second method corresponding to microorganisms having a second size smaller than the first size.

4. The computer program according to any one of claims 1 to 3,

wherein the plurality of methods include a first method for detecting microorganisms having a first size using an object detection model and a second method for detecting microorganisms having a second size smaller than the first size by detecting a moving object from the time-series image.

5. The computer program according to claim 4,

wherein the image is configured by a plurality of frames in chronological order, and in the second method, the moving object is detected based on a difference between each frame and an average image obtained by averaging a plurality of frames over a predetermined period of time.

6. The computer program according to any one of claims 1 to 5, wherein the image is a sludge image obtained by imaging sludge in a wastewater treatment system using an activated sludge method.

7. The computer program according to claim 6,

wherein protozoa as the microorganisms in the sludge image are recognized using the plurality of methods, and a condition of activated sludge is estimated based on conditions of the recognized protozoa in the sludge image.

8. The computer program according to claim 7, the program causing the computer to further execute processing of: deriving proposal information regarding operating conditions of the wastewater treatment system based on the estimated condition of the activated sludge.

9. An information processing method in which a computer executes processing of:

acquiring an image in which a plurality of microorganisms are captured; and recognizing microorganisms in the image by applying a plurality of methods corresponding to types or sizes of the microorganisms to the acquired image.

10. An information processing apparatus, comprising: a control unit that executes processing of acquiring an image in which a plurality of microorganisms are captured and recognizing microorganisms in the image by applying a plurality of methods corresponding to types or sizes of the microorganisms to the acquired image.

FIG. 1

100

FIG. 2

Information Processing Apparatus 1

Control Unit 11

Communication Unit 13

Storage Unit 12

Program 1P

Estimation Data 121

1A

FIG. 3

FIG. 4

Sludge Image

↓

Detection
Model ~ 1 M

↓

Mask Image

Number of Large-Sized Protozoa
　　Colpoda : ○○
　　Paramecium : ○○
　　Rotifers : ○○
　　・・・

Number of Large-Sized Protozoa
Colpoda : Not Appropriate (Increase)
Paramecium : Not Appropriate (Increase)
Rotifers : Appropriate
・・・

Health Condition of Activated Sludge
Poor (Increase in Ammonia Concentration,
Decrease in Dissolved Oxygen Concentration)

FIG. 5

Sludge Image

Frame n

Frame 2

Frame 1

t

Average Image

Frame 1 — Average Image = Moving Object

Frame n — Average Image = Moving Object

Number of Small-Sized Protozoa: ○○

Number of Small-Sized Protozoa: Appropriate

Health Condition of Activated Sludge: Good

## FIG. 6

Sludge Image

Binarized Image

Extract Blob That Satisfy Extraction Condition

⇩

Number of Filamentous Bacteria：○○、Area of Filamentous Bacteria：○○

⇩

Number of Filamentous Bacteria：Appropriate、Area of Filamentous Bacteria：Appropriate

⇩

Health Condition of Activated Sludge：Good

# FIG. 7

Sludge Image

Binarized Image

Feature Quantity

|  | Region 1 | Region 2 | Region 3 | ... | Average Value |
|---|---|---|---|---|---|
| Area | 300 | 1000 | 400 | ... | 770 |

Size of The Flocs : Not Appropriate(Dispersing or Disintegrating)

Health Condition of Activated Sludge :
Poor ( Increase in Sludge Loading , Over-Aeration)

FIG. 8

```
                    ┌─────────────┐
                    │    Start    │
                    └─────────────┘
                           │
                           │                    S11
        ┌──────────────────────────────────────────┐
        │           Receive sludge image           │
        └──────────────────────────────────────────┘
                           │
                           │                    S12
        ┌──────────────────────────────────────────┐
        │      Analyze condition of large-sized     │
        │         protozoa using first method       │
        └──────────────────────────────────────────┘
                           │
                           │                    S13
        ┌──────────────────────────────────────────┐
        │      Analyze condition of small-sized     │
        │        protozoa using second method       │
        └──────────────────────────────────────────┘
                           │
                           │                    S14
        ┌──────────────────────────────────────────┐
        │      Analyze condition of filamentous     │
        │         bacteria using third method       │
        └──────────────────────────────────────────┘
                           │
                           │                    S15
        ┌──────────────────────────────────────────┐
        │    Analyze condition of flocs using fourth method │
        └──────────────────────────────────────────┘
                           │
                           │                    S16
        ┌──────────────────────────────────────────┐
        │   Estimate health condition of activated sludge │
        └──────────────────────────────────────────┘
                           │
                           │                    S17
        ┌──────────────────────────────────────────┐
        │          Derive proposal information       │
        └──────────────────────────────────────────┘
                           │
                           │                    S18
        ┌──────────────────────────────────────────┐
        │     Generate screen information indicating │
        │   estimation result and proposal information │
        └──────────────────────────────────────────┘
                           │
                           │                    S19
        ┌──────────────────────────────────────────┐
        │     Transmit screen information indicating │
        │   estimation result and proposal information │
        └──────────────────────────────────────────┘
                           │
                    ┌─────────────┐
                    │     End     │
                    └─────────────┘
```

# FIG. 9

EP 4 755 855 A1

**Wastewater Management Service**     👤 OO OO

_240_

Wastewater Treatment System ID：OO1

| Microorganism | Condition of Microorganism | | Health Condition of Activated Sludge | Proposal Information |
|---|---|---|---|---|
| Protozoa（1OO μm〜） | Colpoda | Increase <br> Display Image | Poor <br> (Increase in Ammonia Concentration) | Dilute wastewater |
| | Paramecium | Increase <br> Display Image | Poor <br> (Decrease in Dissolved Oxygen Concentration) | Increase in aeration flow rate |
| | Rotifers | Appropriate | Good | — |
| Protozoa（〜1OO μm） | Appropriate | | Good | — |
| Filamentous Bacteria | Appropriate | | Good | — |
| Flocs | Dispersing or Disintegrating <br> Display Image | | poor <br> ( Increase in Sludge Loading , Over-Aeration) | Increase the amount of addition of preparation A <br> Suppression of treatment flow rate |

241   241   242

243

## INTERNATIONAL SEARCH REPORT

| International application No. |
| --- |
| **PCT/JP2024/027376** |

| A. | CLASSIFICATION OF SUBJECT MATTER |
| --- | --- |

*C02F 3/12*(2023.01)i; *C12M 1/34*(2006.01)i; *G06Q 50/00*(2024.01)i; *G06T 7/60*(2017.01)i; *G06T 7/254*(2017.01)i
FI: C02F3/12 H; C02F3/12 P; G06T7/254 A; C12M1/34 D; G06Q50/00; G06T7/60 110

According to International Patent Classification (IPC) or to both national classification and IPC

| B. | FIELDS SEARCHED |
| --- | --- |

Minimum documentation searched (classification system followed by classification symbols)

C02F3/12; C12M1/00-3/10; G06Q50/00-50/20; G06Q50/26-99/00; G16Z99/00; G06T7/00-7/90

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2017/030138 A1 (TORAY INDUSTRIES, INC.) 23 February 2017 (2017-02-23) claims, paragraphs [0035]-[0058] | 1-7, 9-10 |
| Y | claims, paragraphs [0035]-[0058] | 4-5, 8 |
| X | JP 2022-046281 A (JFE ENGINEERING CORPORATION) 23 March 2022 (2022-03-23) claims, paragraphs [0036]-[0049] | 1-3, 6-10 |
| Y | claims, paragraphs [0036]-[0049] | 4-5, 8 |
| P, X | JP 2024-076364 A (KABUSHIKI KAISHA TOSHIBA) 05 June 2024 (2024-06-05) claims, paragraphs [0020]-[0061] | 1-10 |
| A | JP 8-197084 A (KABUSHIKI KAISHA MEIDENSHA) 06 August 1996 (1996-08-06) claims, examples | 1-10 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **26 September 2024** | **08 October 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2024/027376**

| Patent document cited in search report | Publication date (day/month/year) | Patent family member(s) | Publication date (day/month/year) |
|---|---|---|---|
| WO 2017/030138 A1 | 23 February 2017 | (Family: none) | |
| JP 2022-046281 A | 23 March 2022 | (Family: none) | |
| JP 2024-076364 A | 05 June 2024 | (Family: none) | |
| JP 8-197084 A | 06 August 1996 | (Family: none) | |

Form PCT/ISA/210 (patent family annex) (July 2022)

**EP 4 755 855 A1**

**REFERENCES CITED IN THE DESCRIPTION**

**Patent documents cited in the description**

- JP 7172302 B